# EUROPEAN PATENT SPECIFICATION

(11) **EP 0 310 849 B1**
(45) Date of publication and mention of the grant of the patent: **03.03.1993**
(21) Application number: 88115302.7
(22) Date of filing: 19.09.1988
(51) Int. Cl.: C07D 455/04, C07D 405/06

(54) **Process for the synthesis of a benzo [ij] quinolizine-2-carboxylic acid derivative**
Verfahren zur Synthese eines Benzo[ij]chinolizin-2-carbonsäure-Derivates
Procédé pour la synthèse d'un dérivé de l'acide benzo[ij]quinolizine-2-carboxylique

(30) Priority: 05.10.1987 IT 2214287
(43) Date of publication of application: 12.04.1989
(73) Proprietor: S P A SOCIETA' PRODOTTI ANTIBIOTICI S.p.a., 20143 Milano (IT)
(72) Inventor: Bruzzese Tiberio, I-20146 Milano (IT); Signorini, Massimo, I-20144 Milano (IT); Rognoni, Marco, I-20098 San Giuliano Milanese (MI) (IT)
(74) Representative: Giambrocono, Alfonso, Dr. Ing.

(56) References cited:
- GB-A- 2 069 498
- PATENT ABSTRACTS OF JAPAN, Unexamined Applications, C field, vol. 6, no. 57 (C-98) [935], April 14, 1982, page 137 C 98
- MONATSHEFT FUER CHEMIE, vol. 98, 1967, pp.564-578; C.A. BIHLMAYER et al.: "Oxy- und Aminomethylenmeldrumsäuren zur Kenntnis cyclischer Acylale, 17. MiH"

## Description

The object of the present invention is a novel and advantageous method for preparing 6,7-dihydro-9-fluoro-5-methyl-1-oxo-1H,5H-benzo [ij] quinolizine-2-carboxylic acid (flumequine) of formula (I) which is an effective, synthetic antimicrobial agent of the class of quinolone derivatives.

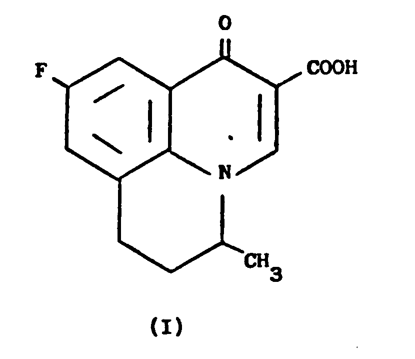

The route known so far for the synthesis of flumequine (e. g., U.S. Patents 3,896,131 - 3,969,463 - 3,985,882 - 4,001,243 - 4,014,877) comprises the following reaction sequence 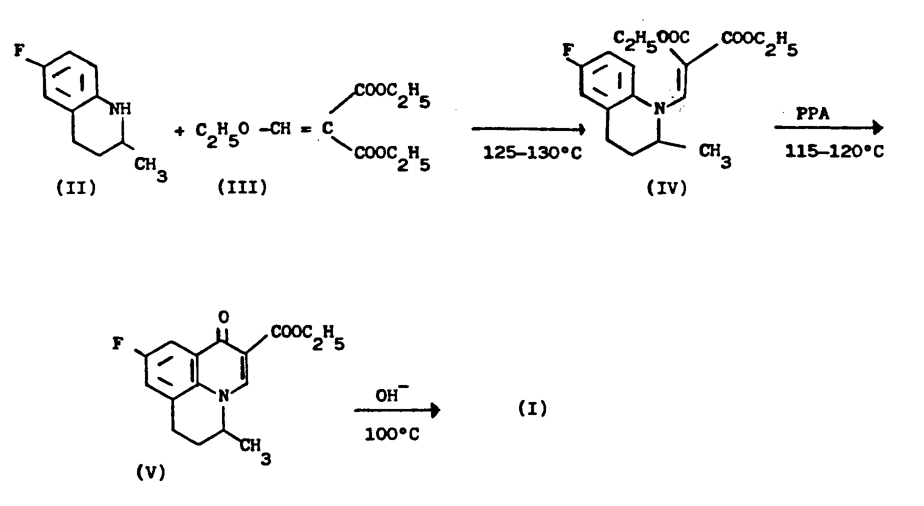
6-fluoro-2-methyl-1,2,3,4-tetrahydroquinoline (II) is reacted with diethyl ethoxymethylene malonate (III) for 3 hours at 125-130°C, whereby the derivative (IV) is obtained. The derivative (IV), when heated for 1 hours at 115-120°C in the presence of polyphosphoric acid (PPA), gives flumequine ethyl ester (V). The saponification of the ester, under hot basic conditions, gives flumequine (I) which is eventually crystallized from dimethylformamide. The intermediate compound (III), in its turn, can be prepared with yields not over 60% by heating for 13 hours at 102-155°C a mixture of diethylmalonate, ethyl orthoformate and acetic anhydride and then fractionating the reaction mixture under vacuum, according to a method known in the literature (Org. Synth. Coll. Vol. III, 395/1955).

It can be easily observed that the above reaction sequence involves the heating of the intermediate compounds for many hours on the whole, at temperatures between 115° and 130°C. Moreover, the resulting ethyl ester has to be further heated at reflux under basic conditions for obtaining the final desired compound.

The intermediate compound (IV) does not need to be isolated, but this advantage is lost when one has anyhow to separate the ester (V) and transfer it into a stainless-steel reactor for the hydrolysis under hot alkaline conditions which cannot be carried out in the reactor (enameled) suitable for the two preceding steps, under neutral and acidic conditions respectively, of the process. Moreover, the need of a long heating under chemically aggressive conditions affects the quality of the final product which has to be crystallized from a high-boiling solvent, such as dimethylformamide, which is difficult to be completely removed, in order to obtain a convenient purity degree (U.S. Pat-.3,896,131).

In GB A-2.069.498, a multiple step process for the preparation of flumequine is described wherein a dialkyl - alkoxymethylene malonate is implied in the condensation reaction with 6-fluoro-2 methyl-1.2.3.4 tetrahydroquinoline (II). As in the previous case, the reaction sequence involves the heating of the intermediate compounds for many hours on the whole, at a temperature of 125°C; further heating at reflux under basic conditions has to be conducted to obtain the final desired compound. Shortcomings are to be recognised in the length of the whole multiple step process and in the low overall yield. A further but shorter multiple step process to obtain flumequine is described in JP-A-572.285 wherein benzoheterocyclic derivatives and cycloisopropylidenyl -analogouses of III are used; purification and isolation are however to be conducted to obtain the final product.

According to the invention, a more favourable result as far as yield, product purity, mild reaction conditions and easy operativeness are concerned, is obtained by reacting 6-fluoro-2-methyl-1,2,3,4-tetrahydroquinoline (II) at room temperature in the presence of p-toluenesulfonic acid as catalyst with a 0.2-1 molar excess of an alkyl orthoformate (IX), wherein R₃ represents a C₁ -C₄ alkyl, and in equimolar amount with a 2,2-disubstituited 1,3-dioxan-4,6-dione (VIII), wherein each R₁ and R₂ , which can be the same or different, represents a hydrogen atom, a branched - or straight-chain C₁ -C₆ alkyl or a phenyl, or R₁ and R₂ together represent a polymethylene group (-CH₂)ₙ -, n being 4 or 5, whereby to give a 2,2 disubstituted 5-[1-(6-fluoro-2-methyl-1,2,3,4-tetrahydroquinolyl] -methylene-1,3-dioxan-4,6-dione of formula (VII);
then said compound of formula (VII) is reacted in the presence of polyphosphoric acid or ethyl polyphosphate to form directly the desired compound (I), which is isolated according to known methods.

The method according to the invention can be summarized as follows: 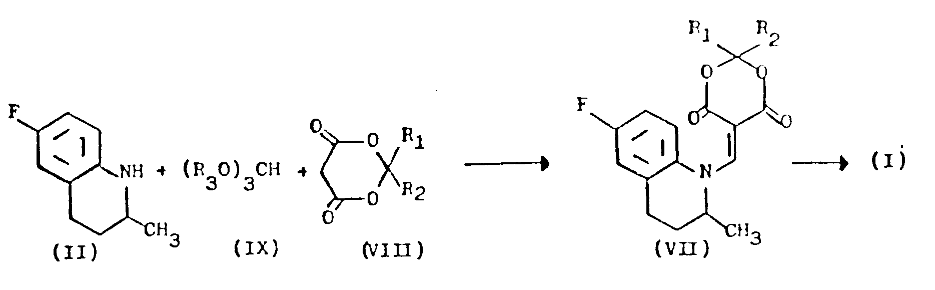
Amine (II) and orthoformates (IX) are commercial products; the 1,3-dioxan-4,6-dione derivatives (VIII) are commercial products or can be easily prepared according to methods known in the literature from malonic acid and carbonyl derivatives R₁-CO-R₂ in acetic anhydride, in the presence of catalytic amounts of concentrated sulfuric acid (e.g.,J.A.C.S., 70, 3426/1948 and Chem.Ber., 94, 929/1961).

The conversion of (VII) to (I) is carried out in the presence of 1-10 (preferably 2) parts by weight of polyphosphoric acid (PPA) at temperatures between 80° and 135°C (preferably 110-115°C), while preferably distilling out of the reaction mixture the resulting carbonyl product R₁-CO-R₂. If this latter boils at a temperature higher than the reaction temperature, the distillation will be advantageously carried out under vacuum. According to a further embodiment of the method, the reaction between (VII) and polyphosphoric acid in the above-mentioned ratios is carried out in the presence of a solvent, such as benzene, toluene, xylene, at temperatures in the range between 80°C and the boiling point of the solvent. The reaction times are in the range between 30 and 180 minutes (preferably 60 minutes).

As condensation agent in this step, the ethyl polyphosphate (PPE) is as much suitable as polyphosphoric acid (PPA).

According to the process of the present invention, flumequine is formed directly by the reaction and can be isolated by cooling to 40°C the reaction mixture, diluting with water, cooling to 10°C and separating by filtration the precipitated product.

Alternatively, the reaction mixture is cooled to about room temperature, aqueous sodium hydroxide is added up to lightly basic pH (8.5-9), the alkaline solution is washed with an hydrocarbon (benzene, toluene, xylene), chlorinated or ethereal solvent, the organic phases are discarded, the aqueous phase is acidified with about 10% HCl, cooled to 10°C and the precipitated product is filtered.

Flumequine obtained according to the process of the present invention is pure enough as to need no further treatment or purification.

The process of the present invention for preparing flumequine (I), presents the following advantages over the prior art:
- extremely mild reaction conditions, easy operativeness and high yields in the preparation of the intermediate compound (VII) from 6-fluoro-2-methyl-1,2,3,4-tetrahydroquinoline (II).
- direct preparation of flumequine from intermediate compound (VII) in a one-step process, which avoids intermediate compounds and is carried out through short heating phases (preferably 60 minutes).

Consequently, the method of the present invention allows significant savings to be obtained, as far as equipments, energy, labour and time are concerned:
- high yield of flumequine as to the starting reagents and the intermediate compound (VII),
- high purity of the resulting products, so that further treatments and purifications are not necessary.

The following examples are intended to illustrate the invention, without however being limitative;

### Example N.1a

### Preparation of 5- 1-(6-fluoro-2-methyl-1,2,3,4-tetrahydroquinolyl)] -methylene-2,2-dimethyl-1,3-dioxan-4,6-dione

2 g (13.9 mmole) of 2,2-dimethyl-1,3-dioxan -4,6-dione, 2,28 g (13.9 mmole) of 6-fluoro-2-methyl-1,2,3,4-tetrahydroquinoline and 2,28 ml (20.6 mmole) of methyl orthoformate are dissolved in 6ml of tetrahydrofurane with 0,2g of p-toluenesulfonic acid. The solution is kept under stirring at room temperature, whereby after 15 minutes a precipitate begins to form. When the reaction is completed (3 hours) an equal volume of ethyl ether is added to the suspension in order to complete the precipitation of the product, the mixture is cooled to 0°C and the white precipitate is filtered.
m.p.: 165°C
Yield: 3.99 g

### Example N. 1b

### Flumequine preparation

3.99 g (12.5 mmole) of 5-[1-(6-fluoro-2-methyl -1,2,3,4-tetrahydroquinolyl)] -methylene-2,2-dimethyl-1,3-dioxan-4,6-dione, 8g of polyphosphoric acid and 20 ml of xylene are heated to 110-115°C under vigorous stirring. After 1 hour of heating under stirring, the reaction mixture is cooled to about room temperature and 10% aqueous sodium hydroxide is added up to pH 8.5-9.

The mixture is allowed to return to room temperature under stirring, the organic layer is separated and the aqueous alkaline solution is washed a few times with ethyl ether. The alkaline solution is cooled in an ice-bath and acidified to pH 3 with concentrated HCl. The resulting precipitate is filtered and washed with water.
Yield: 3.05 g
m.p.: 253-5°C

The product is identical (m.p., IR, TLC) with a true flumequine sample and is pure enough so that further treatments are not necessary.

### Example N. 2a

### Preparation of 5- [1-(6-fluoro-2-methyl-1,2,3,4-tetrahydroquinolyl)]-methylene-2-ethyl-2- methyl-1,3-dioxan-4,6-dione.

The product is prepared according to the procedure of Example 1a by reacting 5g (31.6 mmole) of 2-ethyl-2-methyl-1,3-dioxan-4,6-dione, 5.2 g (31.6 mmole) of 6-fluoro-2-methyl-1,2,3,4-tetrahydroquinoline and 5.12 ml (46.8 mmole) of methyl orthoformate in 15 ml of tetrahydrofurane, in the presence of 0,5g of p-toluenesulfonic acid.
Yield: 9,64g
m.p.: 152°C

### Example N. 2b

### Flumequine preparation

9.5g (28.5 mmole) of the product of Example 2a and 18g of polyphosphoric acid are kept under stirring for 1 hour at 110-115°C. The reaction mixture is cooled to about 40°C, 80 ml of water are added and stirred for 1 hour at 0°C.

The flumequine precipitated from the mixture is filtered and washed with water to neutrality.
Yield: 6.78g
m.p.: 252-4°C

### Example N. 3a

### Preparation of 5- [ 1-(6-fluoro-2-methyl-1,2,3,4-tetrahydroquinolyl)]-methylene-2,2-tetramethylene-1,3-dioxan-4,6-dione

The product is prepared according to the procedure of Example 1a, starting from 4,5 g (26.4 mmole) of 2,2-tetramethylene-1,3-dioxan-4,6-dione, 4,37g (26,4 mmole) of 6-fluoro-2-methyl-1,2,3,4-tetrahydroquinoline and 4,35 ml (39.6 mmole) of methyl orthoformate in 12 ml of tetrahydrofurane, in the presence of 0,4 g of p-toluenesulfonic acid.
Yield: 8,25 g
m.p.: 172-5°C

### Example N. 3b

### Flumequine preparation

Flumequine is prepared according to the procedure of Example 1b, starting from 8g (23.16 mmole) of the product of Example 3a and 16g of polyphosphoric acid in 40ml of xylene.
Yield: 5.6 g
m.p.: 253-5°C

### Example N. 4a

### Preparation of 5-[1-(6-fluoro-2-methyl-1,2,3,4-tetrahydroquinolyl)]-methylene-2-phenyl-1,3-dioxan-4,6-dione

The product is prepared by reacting at room temperature for 30 hours under stirring 2.5 g (13 mmole) of 2-phenyl-1,3-dioxan-4,6-dione, 2.15 g (13 mmole) of 6-fluoro-2-methyl-1,2,3,4-tetrahydroquinoline and 2.1 ml (19 mmole) of methyl orthoformate in 7 ml of tetrahydrofurane in the presence of 0.25 g of p-toluenesulfonic acid. When the reaction is completed the precipitate is filtered.
Yield: 4.05g
m.p. 152-5°C

### Example N. 4b

### Flumequine preparation

Flumequine is prepared by heating under stirring at 110-115°C for a few hours a mixture of 3.8g (10.3 mmole) of the product of Example 4a, 7.5 g of polyphosphoric acid and 20 ml of xylene. The mixture is then treated according to the procedure of Example 1b.
Yield: 1.7g
m.p.: 253-254°C

### Example N. 5a

### Preparation of 5- [1-(6-fluoro-2-methyl-1,2,3,4-tetrahydroquinolyl)]-methylene-2-phenyl-2-methyl-1,3-dioxan-4,6-dione

The product is prepared according to the procedure of Example 1a, starting from 10 g (48.5 mmole) of 2-phenyl-2-methyl-1,3-dioxan-4,6-dione, 8.01 g (48.6 mmole) of 6-fluoro-2-methyl-1,2,3,4-tetrahydroquinoline and 7.9 ml (72,7 mmole) of methyl orthoformate in 25 ml of tetrahydrofurane, in the presence of 0.5 g of p-toluenesulfonic acid.
Yield: 13.9 g
m.p.: 159-160°C.

### Example N. 5b

### Flumequine preparation

Flumequine is prepared by heating at 110-115°C under stiring for 3 hours a mixture of 5g (13.1 mmole) of the product of Example 5a, 10 g of polyphosphoric acid and 25 ml of xylene. The mixture is then treated according to the procedure of Example 1b.
Yield: 2,5 g
m.p.: 253-4°C

### Example N. 6

### Preparation of 5- [1-(6-fluoro-2-methyl-1,2,3,4-tetrahydroquinolyl) ] -methylene-2,2-dimethyl-1,3-dioxan-4,6-dione

1.65 g (10 mmole) of 6-fluoro-2-methyl-1,2-3,4-tetrahydroquinoline and 2,05 g (11 mmole) of 2,2-dimethyl-5-methoxymethylene-1,3-dioxan-4,6-dione [prepared according to Monatshafte für Chemie, 98, 565 (1967)] are reached under stirring and heating at 100-110°C for 1 hour. The cooled reaction mass is taken up in tetrahydrofurane and the solids are filtered and dried.
Yield: 2.5 g
m.p.: 163-5°C

Flumequine is prepared from the resulting product according to the procedure of Example 1b.

## Claims

1. Process for preparing 6,7-dihydro-9-fluoro-5-methyl-1-oxo-1H,5H-benzo[ij]quinolizine-2-carboxylic acid (flumequine, I) 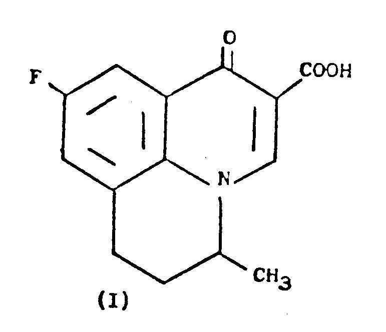 characterized in that 6-fluoro-2-methyl-1,2,3,4-tetrahydroquinoline (II) is reacted at room temperature in the presence of p-toluenesulfonic acid as catalyst with a 0.2-1 molar excess of an alkyl orthoformate (IX), wherein R₃ represents a C₁-C₄ alkyl, and in equimolar amount with a 2,2-disubstituited 1,3-dioxan-4,6-dione (VIII), wherein each R₁ and R₂, which can be the same or different, represents a hydrogen atom, a branched - or straight-chain C₁-C₆ alkyl or a phenyl, or R₁ and R₂ together represent a polymethylene group (-CH₂)ₙ-, n being 4 or 5, whereby to give a 2,2-disubstituted 5- [1-(6-fluoro-2-methyl-1,2,3,4-tetrahydroquinolyl] -methylene-1,3-dioxan-4,6-dione of formula (VII): 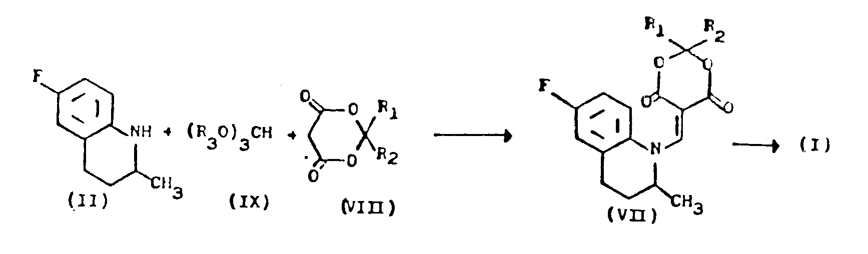 and then said compound of formula (VII) is reacted in the presence of polyphosphoric acid or ethyl polyphosphate to form directly the desired compound (I), which is isolated according to known methods.

2. Process according to claim 1, characterized in that R₃ (IX) and R₁ ,R₂ (VIII) are methyl groups.

## Patentansprüche

1. Verfahren zur Herstellung von 6,7-Dihydro-9-fluor-5-methyl-1-oxo-1H,5H-benzo[ij]chinolizin-2-carbonsäure (Flumequin, I) 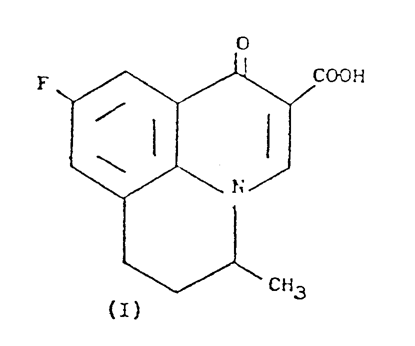 dadurch **gekennzeichnet,** daß 6-Fluor-2-methyl-1,2,3,4-tetrahydrochinolin (II) bei Raumtemperatur in Anwesenheit von p-Toluolsulfonsäure als Katalysator mit einem 0,2-1 molaren Überschuß eines Alkylorthoformiats (IX), worin R₃ eine C₁-C₄-Alkylgruppe bedeutet, und in äquimolarer Menge mit einem 2,2-disubstituierten 1,3-Dioxan-4,6-dion (VIII), worin die Substituenten R₁ und R₂, die gleich oder unterschiedlich sein können, ein Wasserstoffatom, ein verzweigtes oder geradkettiges C₁-C₆-Alkyl oder ein Phenyl bedeuten oder worin R₁ und R₂ zusammen eine Polymethylengruppe (-CH₂)ₙ- bedeuten, worin n für 4 oder 5 steht, umgesetzt werden, wobei ein 2,2-disubstituiertes 5-[1-(6-Fluor-2-methyl-1,2,3,4-tetrahydrochinolyl]-methylen-1,3-dioxan-4,6-dion der Formel (VII): 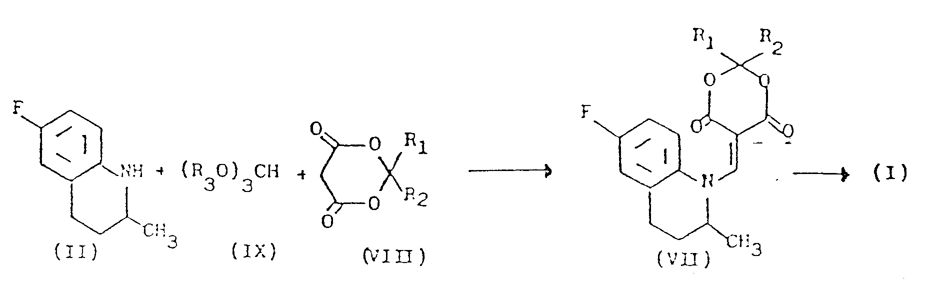 erhalten wird, und dann die Verbindung der Formel (VII) in Anwesenheit von Polyphosphorsäure oder Ethylpolyphosphat unter direkter Bildung der gewünschten Verbindung (I) umgesetzt wird, die nach an sich bekannten Verfahren isoliert wird.

2. Verfahren nach Anspruch 1, dadurch **gekennzeichnet,** daß R₃ (IX) und R₁, R₂ (VIII) Methylgruppen bedeuten.

## Revendications

1. Procédé pour préparer de l'acide 6,7-dihydro-9-fluoro-5-méthyl-1-oxo-1H,5H-benzo|ij|quinolizine-2-carboxylique (flumequine, I) 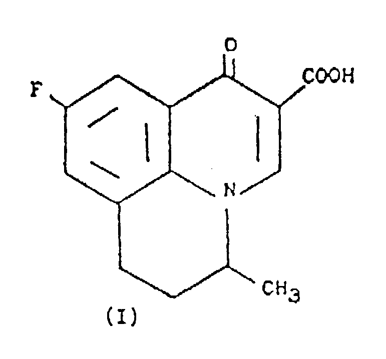 caractérisé en ce que le 6-fluoro-2-méthyl-1,2,3,4-tétrahydroquinoline (II) réagit à température ambiante en présence d'acide p-toluénesulfonique comme catalyseur avec un excès molaire 0,2-1 d'un alkyl orthoformate (IX), où R₃ représente un alkyle C₁-C₄, et en quantité équimolaire avec un 2,2-disubstitué 1,3-dioxan-4,6-dione (VIII), où chaque R₁ et R₂, qui peut être identique ou différent, représente un atome d'hydrogène, un alkyl C₁-C₆ ramifié ou à chaîne rectiligne ou un phényl, ou R₁ et R₂ représentent ensemble un groupe polyméthylène (-CH₂)ₙ-, n étant 4 ou 5, de façon à donner un 2,2-disubstitué 5-|1-(6-fluoro-2-méthyl-1,2,3,4-tétrahydroquinolyl|-méthylène-1,3-dioxan-4,6-dione de formule (VII); 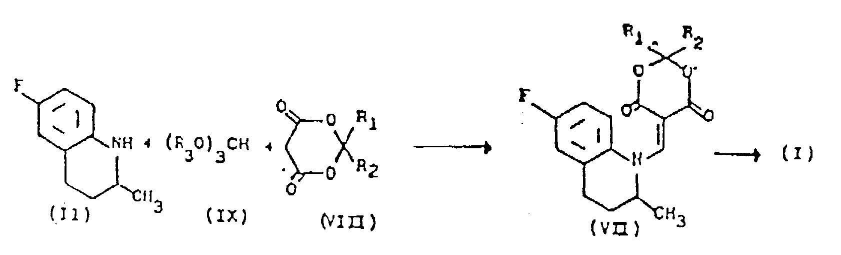 et ce composé de la formule (VII) réagit en présence d'acide polyphosphorique ou d'éthyl polyphosphate pour former directement le composé désiré (I), qui est isolé selon des méthodes connues.

2. Procédé conforme à la revendication 1, caractérisé en ce que R₃ (IX) et R₁,R₂ (VIII) sont des groupes méthyl.
